# EUROPÄISCHE PATENTSCHRIFT

(11) **EP 2 383 398 B1**
(45) Veröffentlichungstag und Bekanntmachung des Hinweises auf die Patenterteilung: **12.08.2015**
(21) Anmeldenummer: 11003041.8
(22) Anmeldetag: 12.04.2011
(51) Int. Cl.: E03F 9/00, A61L 9/01

(54) **Verfahren zur Bekämpfung von üblen Gerüchen**
Method for fighting strong odours
Procédé de lutte contre des odeurs nauséabondes

(30) Priorität: 29.04.2010 DE 102010018719
(43) Veröffentlichungstag der Anmeldung: 02.11.2011
(73) Patentinhaber: I Tech OHG, 58455 Witten (DE)
(72) Erfinder: Rademacher, Franziska, 45525 Hattingen (DE); Hüggenberg, Udo, 45525 Hattingen (DE)
(74) Vertreter: Behrendt, Arne

(56) Entgegenhaltungen:
- DE-C1- 3 726 636
- DE-U1- 20 307 502

## Beschreibung

Die Erfindung betrifft ein Verfahren zur Bekämpfung des üblen Geruchs von Abluft aus Behältern oder Räumen, die Fäkalien, organische Abfälle oder dergleichen enthalten, bei welchem in die Abluft ein Fluid in fein verteilter Form eingebracht wird, welches den Geruch der in der Abluft enthaltenen Geruchsstoffe neutralisiert.

Ein derartiges Verfahren ist beispielsweise aus der DE 37 26 636 C1 bekannt. Das dort verwendete Fluid enthält als Desodorantien-Zinkricinoleat mit Zinkverbindungen von mehrfach hydroxilierten höheren Fettsäuren, Oxaminen und Harnsäuren. Weiterhin enthält das dort verwendete Fluid als Lösungsmittel Wasser und ein- oder mehrwertige Alkohole, insbesondere Ethanol, Isopropylalkohol, Propylenglycol, Dipropylenglykol, Glycerin und Trimethylolpropan. Schließlich enthält das dort verwendete Fluid Lösungsvermittler, z. B. in Form von hydrolysierten Enadukten und Diels-Alder-Addukten von Ricenfettsäuren und Maleinsäureanhydrid. Ein Fluid aus solchen Substanzen ist wegen seiner Inhaltsstoffe kostspielig und umwelttechnisch problematisch, weil einige Inhaltsstoffe reaktiv und damit aggressiv sind oder sich nach Gebrauch nicht restlos verflüchtigen, was für die Umweltverträglichkeit ungünstig ist.

Ein ähnliches Verfahren ist beispielsweise aus der DE 203 07 502 U1 bekannt und wird dort zur Unterdrückung der Geruchsbelästigung durch Abluft aus dem Tank eines Saugfüllfahrzeugs verwendet, welches z. B. für den Abtransport von Fäkalien eingesetzt ist. Hier wird in das Abluftrohr des Saugspülfahrzeugs ein Fluid eingesprüht, welches die in der Abluft enthaltenen Geruchsstoffe chemisch und/oder physikalisch neutralisiert. Dabei ist in der DE 203 07 502 U1 offen gelassen worden, welches Fluid verwendet wird. Es wird lediglich ein allgemeiner Hinweis gegeben, dass das geruchsbekämpfende Fluid Substanzen enthalten soll, die den speziellen Geruch der in der Abluft enthaltenen Geruchsstoffe überdecken oder dahingehend chemisch und/oder physikalisch auf diese Geruchsstoffe einwirken, dass sie ihre unangenehme Wirkung verlieren. Dabei sollen die in dem Fluid enthaltenen Wirkstoffe dem jeweiligen Einzelfall angepasst werden.

Es ist Aufgabe der Erfindung, ein Fluid für das oben genannte Verfahren bereitzustellen, welches insbesondere für die Neutralisierung von Gerüchen aus organischen Zersetzungsprozessen geeignet sind, z. B. für Fäkalien und Mist, Schlachtabfälle, Mülldeponien, Kläranlagen, transportable Toilettenanlagen, Leichenhallen etc. Dabei soll das für die Geruchsbekämpfung verwendete Fluid natürlich ein möglichst breites Spektrum an Geruchsstoffen neutralisieren und außerdem ungiftig, umweltverträglich und wenig aggressiv sein.

Gegenstände der Erfindung sind das Verfahren gemäß Patentanspruch 1 und die Verwendung gemäß Patentanspruch 2.

Es hat sich überraschenderweise herausgestellt, dass ein aus Glyzerin in wässriger Lösung hergestellter Nebel hervorragend für die Bekämpfung von üblen Gerüchen geeignet ist, die in der Abluft von organischen Zersetzungsprozessen vorkommen. Dabei ist Glyzerin weitestgehend ungiftig und wenig aggressiv.

Die duftneutralisierende Wirkung von Glyzerin ist wahrscheinlich darauf zurückzuführen, dass Glyzerin in fein verteilter Form zur Veresterung und damit Bindung von in der Abluft enthaltenen übel riechenden Fettsäuren beiträgt und aufgrund seiner stark hygroskopischen Wirkung viel Wasser in Form von kondensierenden Nebeltröpfchen aus der Abluft aufnimmt und dabei Duftstoffe physikalisch an sich bindet.

Obwohl die Glyzerinkonzentration in der wässrigen Lösung nicht von ausschlaggebender Bedeutung ist, hat sich insbesondere unter wirtschaftlichen und handhabungstechnischen Gesichtspunkten ein Glyzeringehalt von 2,5 Vol.-% bis 10 Vol.-% in der wässrigen Lösung bewährt.

Weitere Verbesserungen lassen sich erzielen, wenn das verwendete Fluid zusätzlich zu dem Glyzerin bis zu 2,5 Vol.-% Glykol enthält, welches im Wesentlichen oben angesprochene hygroskopische Wirkung verstärkt.

Schließlich kann das Fluid zusätzlich zu Glyzerin oder zusätzlich zu Glyzerin und Glykol bis zu 2,5 Vol.-% Essigsäure - n-Butylester enthalten. Diese Substanz, die ebenfalls völlig gefahrlos einsetzbar ist, hat einen angenehmen, fruchtartigen Geruch, der nach der Neutralisierung der üblen Gerüche aus der Abluft als einziger wahrnehmbar bleibt.

Das Verfahren gemäß der Erfindung kann im Prinzip überall in Abluftrohren, Abluftkanälen, von Abluft durchströmten Räumen oder über offenen Behältern verwendet werden, aus deren Öffnung Abluft austritt. Das Fluid wird mittels Hochdruckdüsen oder Zweistoffdüsen (Druckluft-Fluid) zu einem feinen Nebel zerstäubt und anschließend - ggf. unter Zuhilfenahme eines Lüfters - mit der Abluft durchmischt. Der aus dem Fluid hergestellte Nebel neutralisiert die in den Abluft enthaltenen Duftstoffe und gibt der Abluft im Ergebnis einen angenehmen fruchtartigen Geruch.

### Beispiel 1:

In einem Schlachthof wurde über einem Fleischabfallbehälter bei dessen Entleerung mittels Zweistoff-Düsen und Ventilator ein fein verteilter Nebel bestehend aus 10 Vol.-% Glyzerin, 2 Vol.-% Glykol, 2 Vol.-% Essigsäure -n-Butylester und 86 Vol.-% Wasser ausgebracht. Dabei wurden die bei dem Entleerungsvorgang normalerweise entstehenden üblen Gerüche vollständig vermieden. Im Ergebnis entstand im Abluftbereich der Behälterentleerung ein fruchtiger, angenehmer Geruch.

### Beispiel 2:

An einem Transportfahrzeug für Fäkalien wurde während des Beladevorgangs des Tanks in das Abluftrohr des Tanks mit Hilfe einer Hochdruck-Sprühdüse ein fein verteilter Nebel aus 10 Vol.-% Glyzerin, 2 Vol.-% Glykol, und 1 Vol.-% Essigsäure-n-Butylester und 87 Vol-% Wasser eingesprüht. Dieser Nebel hat die in der Abluft aus dem Tank enthaltenen Duftstoffe vollständig mitneutralisiert. Spürbar war nur noch ein angenehmer fruchtartiger Geruch nach dem Essigsäure-n-Butylester.

## Patentansprüche

1. Verfahren zur Bekämpfung des üblen Geruchs von Abluft aus Behältern oder Räumen, die Fäkalien, organische Abfälle oder dergleichen enthalten, bei welchem in die Abluft ein Fluid in fein verteilter Form eingebracht wird, welches Wasser und Glycerin enthält und den Geruch der in der Abluft enthaltenen Geruchsstoffe neutralisiert,
**dadurch gekennzeichnet, dass** das Fluid besteht aus:
- 2,5 Vol.-% bis 10 Vol.-% Glyzerin
- bis zu 2,5 Vol.-% Glycol
- bis zu 2,5 Vol.-% Essigsäure-n-Butylester
- Rest: Wasser.

2. Verwendung eines fein verteilten Fluids aus:
- 2,5 Vol.% bis 10 Vol.% Glyzerin
- bis zu 2,5 Vol.-% Glycol
- bis zu 2,5 Vol.-% Essigsäure-n-Butylester
- Rest: Wasser
zur Neutralisierung der üblen Gerüche von Abluft aus Behältern oder Räumen, die Fäkalien, organische Abfälle oder dergleichen enthalten.

## Claims

1. A method of combating the foul odour of discharge air from containers or spaces which contain faeces, organic waste or the like, in which there is introduced into the discharge air a fluid in finely distributed form which contains water and glycerine and neutralises the odour of the odorous substances contained in the discharge air, **characterised in that** the fluid comprises:
- 2.5% by volume to 10% by volume of glycerine,
- up to 2.5% by volume of glycol,
- up to 2.5% by volume of acetic acid n-butyl ester, and
- balance: water.

2. Use of a finely distributed fluid comprising:
- 2.5% by volume to 10% by volume of glycerine,
- up to 2.5% by volume of glycol,
- up to 2.5% by volume of acetic acid n-butyl ester, and
- balance: water
for neutralisation of the foul odours of discharge air from containers or spaces which contain faeces, organic waste or the like.

## Revendications

1. Procédé de lutte contre l'odeur nauséabonde d'air vicié de contenants ou de pièces contenant des matières fécales, des déchets organiques ou des matières analogues, selon lequel procédé un fluide est introduit dans l'air vicié sous une forme finement dispersée, le fluide contenant de l'eau et de la glycérine et neutralisant les particules d'odeur contenues dans l'air vicié,
**caractérisé en ce que** le fluide se compose de :
- 2,5 à 10 % en volume de glycérine,
- jusqu'à 2,5 % en volume de glycol,
- jusqu'à 2,5 % en volume d'ester butylique n d'acide acétique,
- le reste : de l'eau.

2. Utilisation d'un fluide finement dispersé composé de :
- 2,5 à 10 % en volume de glycérine,
- jusqu'à 2,5 % en volume de glycol,
- jusqu'à 2,5 % en volume d'ester butylique n d'acide acétique,
- le reste : de l'eau
pour la neutralisation des odeurs nauséabondes d'air vicié de contenants ou de pièces contenant des matières fécales, des déchets organiques ou des matières analogues.
